# EUROPEAN PATENT APPLICATION

(11) **EP 4 778 444 A1**
(43) Date of publication of application: **22.07.2026**
(21) Application number: 24865454.3
(22) Date of filing: 10.09.2024
(51) Int. Cl.: A61B 3/032, A61B 3/028, G06T 19/00

(54) **INFORMATION PROCESSING SYSTEM, INFORMATION PROCESSING METHOD, AND PROGRAM**

(30) Priority: 13.09.2023 JP 2023148539; 31.01.2024 JP 2024013417; 31.03.2024 JP 2024058400
(71) Applicant: Innojin, Inc., Tokyo 113-0033 (JP); Juntendo Educational Foundation, Tokyo 113-8421 (JP)
(72) Inventor: INOMATA, Takenori, Tokyo 113-0033 (JP); OKUMURA, Yuichi, Tokyo 113-0033 (JP)
(74) Representative: Osterhoff, Utz
(86) International application number: PCT/JP2024/032422
(87) International publication number: WO 2025/057954

(57) **Abstract**

[Technical Problem]

An information processing system that enables easy measurement of visual acuity.

[Solution to Problem]

An information processing system comprising: an image display unit that displays images on each of display devices corresponding to a left eye and a right eye of a user, respectively, to provide a virtual space to the user; an indicator display unit that arranges an indicator and a peripheral object in the virtual space at positions separated from the user, displays the indicator only on the display device corresponding to one of the left eye and the right eye, displays the peripheral object on the display devices corresponding to both of the left eye and the right eye, and moves the indicator to a distance at which the user can visually recognize the indicator; and a visual acuity measurement unit that determines visual acuity of one of the user's left and right eyes according to the distance.

## Description

### Technical Field

The present invention relates to an information processing system, an information processing method, and a program.

### Background Art

PTL 1 discloses a system that displays an indicator in a virtual space to measure visual acuity.

### Citation List

### [Patent Literature

[PTL1] Japanese Patent Application Publication No. 2022-512186

### Summary of Invention

### Technical Problem

However, in the system of PTL 1, only binocular visual acuity can be measured.

The present invention has been made in view of the above circumstances, and an object thereof is to provide a technology that can easily measure visual acuity.

### Solution to Problem

According to one aspect of the present invention, there is provided an information processing system comprising: an image display unit that displays images on each of display devices corresponding to each of left and right eyes of a user to provide a virtual space to the user; an indicator display unit that arranges an indicator and a peripheral object in the virtual space at positions separated from the user, displays the indicator only on the display device corresponding to one of the left and right eyes, displays the peripheral object on the display devices corresponding to both of the left and right eyes, and moves the indicator to a distance at which the user can visually recognize the indicator; and a visual acuity measurement unit that determines visual acuity of one of the user's left and right eyes according to the distance.

Other objects, features, and advantages of the present application will become apparent from the following description of embodiments and the accompanying drawings.

### Advantageous Effects of Invention

According to the present invention, visual acuity can be easily measured.

### [Brief Description of Drawings]

[Fig. 1] Fig. 1 is a diagram showing a configuration example of a visual acuity measurement system.
[Fig. 2] Fig. 2 is a diagram showing a configuration example of HMD 1.
[Fig. 3] Fig. 3 is a diagram showing a software configuration example of computer 2.
[Fig. 4] Fig. 4 is a diagram explaining how an indicator appears.
[Fig. 5] Fig. 5 is a diagram explaining display of a dotted line.
[Fig. 6] Fig. 6 is a diagram explaining operation of computer 2.
[Fig. 7] Fig. 7 is a diagram showing a configuration example of HMD 1 according to a second embodiment.
[Fig. 8] Fig. 8 is a diagram showing a configuration example of computer 2 according to the second embodiment.
[Fig. 9] Fig. 9 is a diagram explaining operation of computer 2 according to the second embodiment.

### First Embodiment

Hereinafter, an information processing system according to one embodiment of the present invention will be described. The information processing system of the present embodiment causes a user to wear a head-mounted display (HMD 1) and is configured to measure the visual acuity of the user in a virtual space. Note that the HMD 1 may be any wearable device that provides a virtual space to the user, and can be, for example, a glasses-type augmented reality (AR) device (glasses-type display). Further, the HMD 1 does not have to be a wearable device, and an autostereoscopic display, for example, can also be adopted.

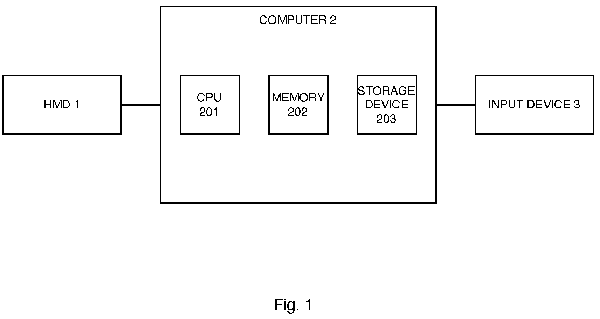

Fig. 1 is a diagram showing a configuration example of a visual acuity measurement system. The visual acuity measurement system of the present embodiment is configured to include a computer 2. The computer 2 is connected to the HMD 1 and an input device 3, can accept input of data from the input device 3, and can control display of images on the HMD 1. As shown in Fig. 1, the computer 2 can include a CPU 201, a memory 202, and a storage device 203. The storage device 203 is, for example, a hard disk drive, a solid state drive, a flash memory, or the like that stores various data and programs. Note that each functional unit of the computer 2, which will be described later, is realized by the CPU 201 reading out a program stored in the storage device 203 to the memory 202 and executing the program, and each storage unit of the computer 2 can be realized as a part of a storage area provided by the memory 202 and the storage device 203.

The input device 3 is, for example, a controller, a microphone, a keyboard, a mouse, a touch panel, or the like. The input device 3 can support the user in performing predetermined input in the virtual space. The input device 3 can be configured, for example, as a set of controllers for the left hand and the right hand. The input device 3 can include, for example, an operation trigger button, an infrared LED, a sensor, a joystick, a menu button, and the like. Further, the input device 3 can detect posture and movement by an acceleration sensor (not shown) or the like, and can input posture and movement data to the computer 2 as input data.

The computer 2 performs a visual acuity examination of the user, which will be described later.

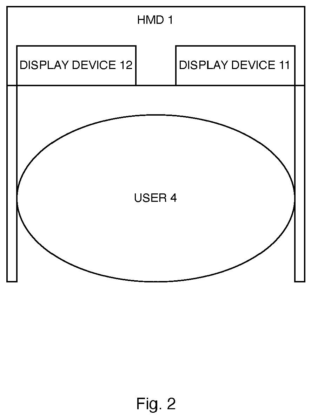

Fig. 2 is a diagram showing a configuration example of the HMD 1. The HMD 1 can be worn on the head of a user 4. The HMD 1 includes display devices 11 and 12 so as to be disposed in front of the left and right eyes of the user 4. The display device 11 displays an image to one eye (the right eye in the example of Fig. 2). The display device 12 displays an image to the other eye (the left eye in the example of Fig. 2). As the display devices 11 and 12, for example, optically transmissive and non-transmissive displays can be adopted. The user 4 can visually recognize the virtual space by simultaneously viewing the images of the display devices 11 and 12 with the left and right eyes.

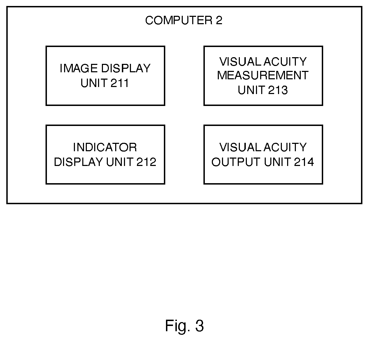

Fig. 3 is a diagram showing a software configuration example of the computer 2. The computer 2 can include an image display unit 211, an indicator display unit 212, a visual acuity measurement unit 213, and a visual acuity output unit 214.

The image display unit 211 provides a virtual space to the user by displaying images on each of the display devices 11 and 12 corresponding to each of the left and right eyes of the user. A known mechanism for stereoscopic vision using a head-mounted display may be adopted.

The indicator display unit 212 arranges an indicator in the virtual space. The indicator display unit 212 arranges the indicator separated from the user in the virtual space. The indicator can be, for example, a Landolt ring. The indicator display unit 212 can arrange the indicator such that a plane on which the indicator such as a Landolt ring is drawn is perpendicular to the line-of-sight direction of the user.

The indicator display unit 212 can generate images to be displayed on each of the display devices 11 and 12 according to the position and size of the indicator arranged in the virtual space, and display the images on the display devices 11 and 12. The indicator display unit 212 displays an image related to the indicator arranged in the virtual space only on either one of the display devices 11 and 12 (the one corresponding to the eye for which visual acuity is to be measured).

The image display unit 211 arranges an object different from the indicator (hereinafter, a peripheral object) in the virtual space. The image display unit 211 arranges the peripheral object so that the user can visually recognize the peripheral object simultaneously with the indicator. The image display unit 211 can arrange the peripheral object within the viewing angle of the user. The image display unit 211 can arrange the peripheral object around the indicator. The peripheral object can be approximately the same size as or larger than the indicator. The peripheral object may be smaller than the indicator. There may be a plurality of peripheral objects. The peripheral object can be any object such as, for example, a window, a shelf, examination equipment, a desk, a book, a painting, or the like. The image display unit 211 displays the peripheral object (an image corresponding to the peripheral object) on both the display devices 11 and 12. Even if the indicator (an image corresponding to the indicator) is displayed only on one of the display devices 11 and 12, by arranging the peripheral object, the user can perform stereoscopic vision by binocular vision, and the state of the eyes and brain performing stereoscopic vision with respect to the virtual space can also be the state of the eyes and brain for which stereoscopic vision has been performed with respect to the indicator displayed only to one eye.

The image display unit 211 can arrange the peripheral object in the peripheral vision of the user. Generally, human eyes are strong against parallax in the peripheral vision, and can maintain a three-dimensional feeling even if the deviation is large. If stereoscopic vision is possible in the peripheral vision, stereoscopic vision is also possible in the central vision. Therefore, by displaying the peripheral object in both eyes in the peripheral vision and arranging the indicator in the central vision, the user can perform stereoscopic vision of the indicator even if the indicator is displayed only to one eye.

The indicator display unit 212 can also arrange a frame around the indicator. The frame can be displayed on both the display devices corresponding to the left and right eyes. When the indicator is displayed only to one eye, it is difficult to perceive depth and achieve focus, but by arranging a frame around the indicator and displaying the frame to both eyes, it is expected that depth can be perceived and focusing becomes easier.

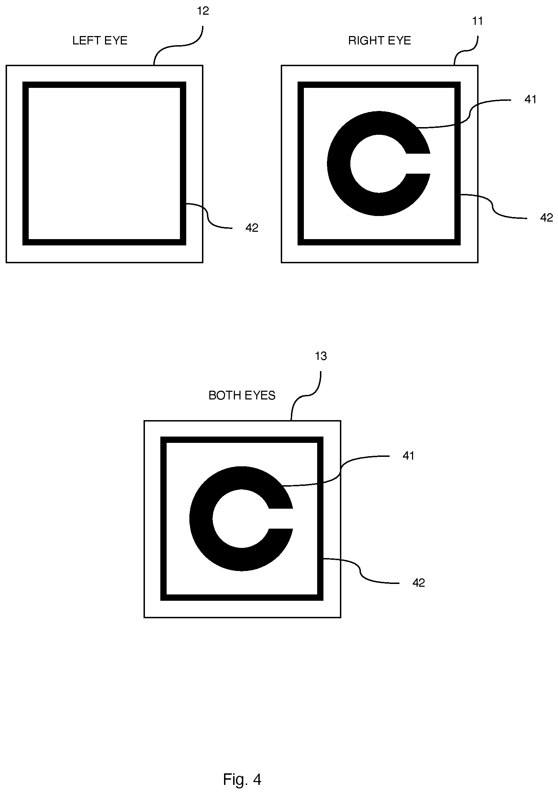

Fig. 4 is a diagram for explaining how the indicator appears. As shown in the figure, an indicator 41 is displayed on the display device 11 in front of the right eye, but the indicator 41 is not displayed on the display device 12 in front of the left eye. However, the indicator 41 is displayed in the binocular vision field of view 13. The indicator 41 is visually recognized only by the right eye. A frame 42 is displayed around the indicator 41. The frame 42 is displayed on both the display devices 11 and 12, and is naturally recognized in the binocular vision field of view 13.

The indicator display unit 212 moves the indicator to a distance at which the user can visually recognize the indicator. The indicator display unit 212 can move the indicator to a distance at which the user can see best. The indicator display unit 212 accepts input as to whether or not the user was able to visually recognize the indicator, and determines the distance at which visual recognition is possible or at which the user can see best. The indicator display unit 212, for example, arranges the indicator at a predetermined distance (which may be a fixed value set in advance or a distance according to the self-reported visual acuity of the user), gradually brings the indicator closer to the user, accepts input that visual recognition has become possible from the user, stops the indicator at the position at that time, and obtains the distance from the position of the stopped indicator to the user. Further, the indicator display unit 212 may move the indicator several times in the front-rear direction from the user, accept designation of the position at which the indicator can be seen best, determine the position of the indicator, and obtain the distance from the determined position to the user. The indicator display unit 212 moves the frame in accordance with the movement of the indicator. The frame is moved such that the distance from the user to the indicator and the distance from the user to the frame are the same (a state in which the frame surrounds the indicator).

The indicator display unit 212 can change the size of the indicator in the virtual space according to the distance such that the size of the indicator visually recognized by the user is constant. The indicator display unit 212 can perform expansion and contraction of the indicator such that if the distance between the user and the indicator is large, the size of the indicator also becomes large, and if the distance between the user and the indicator is small, the size of the indicator also becomes small.

### Spherical Power

The visual acuity measurement unit 213 can determine the spherical power of the eye to be measured based on the distance from the user to the indicator that can be visually recognized. The relationship between the distance and the spherical power can be set, for example, by setting a function in advance. Further, a learning model in which the relationship between the distance from the user to the indicator that can be visually recognized or can be seen best and the spherical power measured using conventional equipment is learned by machine learning is prepared, and the visual acuity measurement unit 213 can also estimate the visual acuity by giving the distance to the learning model.

### Cylindrical Power

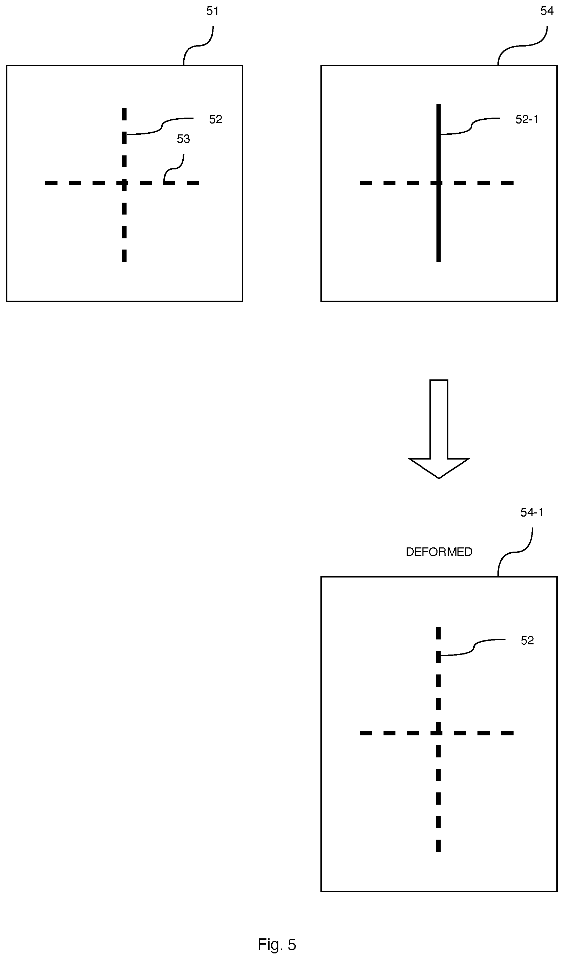

The indicator display unit 212 can display vertical and horizontal dotted lines as an indicator. The dotted lines are also displayed only on either the display device 11 or 12 corresponding to the eye to be examined. Fig. 5 is a diagram for explaining display of dotted lines. The indicator display unit 212 can display, as an indicator, a dotted line 52 (first dotted line) extending in the vertical direction and a dotted line 53 (second dotted line) extending in the horizontal direction. The dotted line 52 and the dotted line 53 are arranged on the plane of the indicator (Landolt ring). In the case of an eye without astigmatism, as in the field of view 51 of Fig. 5, the vertical and horizontal dotted lines 51 and 52 are visually recognized as dotted lines with the same spacing. In the case of astigmatism, as in the field of view 54 of Fig. 5, the dotted line 52 and/or the dotted line 53 appears as a solid line (or the dot spacing is narrow). When either the dotted line 52 or 53 appears as a solid line (or the dot spacing appears narrow) to the user, the indicator display unit 212 deforms the indicator (all dotted lines) so as to enlarge the indicator in the direction of the dotted line that appears as a solid line or the dotted line that appears to have short dot spacing, such that both the dotted lines 52 and 53 appear as dotted lines (dotted lines with the same spacing) to the user. In the example of Fig. 5, in the field of view 54, the vertical dotted line 52-1 is shown as appearing as a solid line. In this case, as shown in the field of view 54-1, by extending in the vertical direction, the dotted line 52 can be visually recognized as a dotted line even by an astigmatic eye.

Note that the indicator display unit 212 may change (lengthen in the example of Fig. 5) the length of the dotted line (the horizontal dotted line 53 in the example of Fig. 5) orthogonal to the direction of expansion and contraction such that the aspect ratio of the indicator is constant (1:1 in the example of Fig. 5).

Further, the indicator display unit 212 can also display dotted lines extending in diagonal directions together with the vertical and horizontal dotted lines. Similarly to the above, the indicator (all dotted lines) can be deformed such that the dotted line that appears as a solid line or the dotted line that appears to have short dot spacing is in the direction of the dotted line, such that all the dotted lines in the vertical, horizontal, and diagonal directions are visually recognized as dotted lines.

The visual acuity measurement unit 213 can determine the cylindrical power of the user according to the degree of deformation of the indicator. The relationship between the degree of deformation of the indicator and the cylindrical power can be set as a function in advance. Further, a learning model in which the relationship between the degree of deformation and the cylindrical power measured using a conventional method is learned by machine learning is prepared, and the visual acuity measurement unit 213 can also estimate the cylindrical power by giving the degree of deformation to the learning model. Note that the relationship between the degree of deformation and the direction of deformation and the cylindrical power may be set by a function or learned by machine learning.

The visual acuity measurement unit 213 can determine the axis of astigmatism based on the direction of deformation.

The visual acuity measurement unit 213 can calculate the equivalent spherical power based on the spherical power and the cylindrical power. The equivalent spherical power can be calculated by adding one-half of the cylindrical power to the spherical power.

The visual acuity output unit 214 outputs the measured visual acuity. The visual acuity output unit can output the spherical power, the cylindrical power, and the axis of astigmatism. The visual acuity output unit 214 can, for example, transmit the measured visual acuity to a terminal operated by an examination technician to display the measured visual acuity. The visual acuity output unit 214 may, for example, print the measured visual acuity from a printer or the like.

### Operation

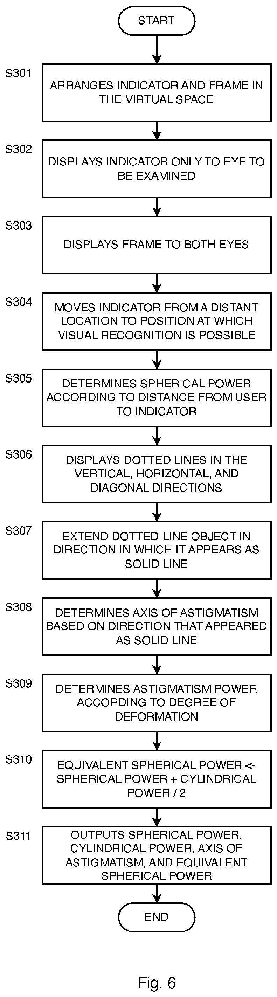

Fig. 6 is a diagram for explaining the operation of the computer 2.

The computer 2 arranges the indicator and frame in the virtual space (S301), displays the indicator only to the eye to be examined (S302), and displays the frame to both eyes (S303). The computer 2 moves the indicator from a distant location to a position at which visual recognition is possible (S304), and determines the spherical power according to the distance from the user to the indicator that can be visually recognized (S305).

The computer 2 arranges vertical, horizontal, and diagonal dotted lines in the virtual space, displays the dotted lines only to the eye to be examined (S306), and causes the user to designate a dotted line that appears as a solid line, and deforms the dotted lines such that all the vertical, horizontal, and diagonal dotted lines are extended in the direction of the designated dotted line (S307). The computer 2 determines the axis of astigmatism based on the direction that appeared as a solid line (S308), and determines the astigmatism power according to the degree of deformation of the indicator (S309).

The computer 2 calculates the equivalent spherical power from the spherical power and the cylindrical power (S310), and outputs the spherical power, the cylindrical power, the axis of astigmatism, and the equivalent spherical power (S311).

As described above, according to the information processing system of the present embodiment, the visual acuity of one of the left and right eyes can be easily measured using the virtual space.

### Second Embodiment

In the second embodiment, lenses for correcting visual acuity are provided in the HMD 1, and corrected visual acuity is measured.

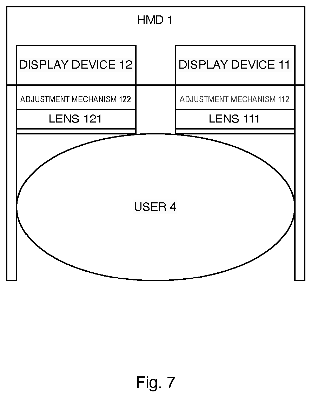

Fig. 7 is a diagram showing a configuration example of the HMD 1 according to the second embodiment. In the second embodiment, the HMD 1 further includes lenses 111 and 121 between the display devices 11 and 12 and the left and right eyes of the user 4 wearing the HMD 1. Further, adjustment mechanisms 112 and 122 for diopter (refractive power) are disposed on the lenses 111 and 121. The adjustment mechanisms 112 and 122 can change the refractive power of each of the left and right eyes of the user 4. The adjustment mechanisms 112 and 122 can, for example, change the refractive power from 0D to 7D. The adjustment mechanisms 112 and 122 can change the refractive power by, for example, changing the positions of the lenses 111 and 121 between the display devices 11 and 12 and the eyes of the user 4. Note that the adjustment mechanisms 112 and 122 may be mechanisms that allow the lenses 112 and 121 to be replaced. Note that the refractive power may be changed by arranging lenses for astigmatism between the lenses 112 and 121 and the display devices 11 and 12 or the left and right eyes of the user. The adjustment mechanisms 112 and 122 may, for example, include an actuator that changes the refractive power under control from the computer 2, or may allow the user 4 to manually change the refractive power. Further, the adjustment mechanisms 112 and 122 can include a function of responding with the refractive power (for example, 0D to 7D, etc.) in response to a request from the computer 2.

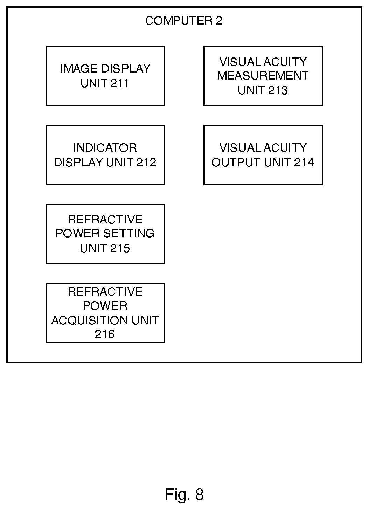

Fig. 8 is a diagram showing a configuration example of the computer 2 according to the second embodiment. In the second embodiment, the computer 2 further includes a refractive power setting unit 215 and a refractive power acquisition unit 216.

The refractive power setting unit 215 sets the refractive power of the HMD 1. The refractive power setting unit 215, for example, accepts input of a refractive power to be set from an examination technician or a user, transmits a refractive power setting request including the refractive power to the adjustment mechanisms 112 and 122 of the HMD 1, and can set the refractive power by, for example, activating an actuator that adjusts the positions of the lenses 111 and/or 121 such that the adjustment mechanisms 112 and 122 achieve the refractive power set in the setting request. Further, the refractive power setting unit 215, for example, accepts input of a refractive power to be set from an examination technician (for example, receives the refractive power from a terminal of the examination technician), outputs a message instructing the user to set the refractive power (for example, can display the message on the display devices 11 and 12 or output the message by voice from a speaker (not shown)), and may cause the user to manually adjust the refractive power. Note that for the refractive power, input of both left and right refractive powers may be accepted and set in each of the adjustment mechanisms 112 and 122, or input of only the refractive power of either the left or right eye to be examined may be accepted, and the refractive power may be set by the above-described method for the adjustment mechanism 112 or 122 corresponding to the eye to be examined. Further, the examination technician may transmit the refractive power from a remote terminal to the computer 2 via a communication network.

The refractive power acquisition unit 216 acquires the refractive power set in the HMD 1. The refractive power acquisition unit 216 can, for example, accept input of the refractive power from an input device such as a keyboard, a touch panel, a mouse, or a microphone. The refractive power acquisition unit 216 may acquire the set refractive power from the adjustment mechanisms 112 and 122. The refractive power acquisition unit 216 can confirm that the refractive power set by the refractive power setting unit 215 and the acquired refractive power match, and can output an alert if they do not match.

The indicator display unit 212 and the visual acuity measurement unit 213 have functions similar to those of the first embodiment, and can measure visual acuity with corrected visual acuity.

Further, the visual acuity measurement unit 213 displays an indicator such as a Landolt ring or a character on the display device 11 or 12, inquires of the user whether the indicator can be seen (the inquiry may be output by voice or may be displayed as a character string or the like on the display devices 11 and 12, or, for example, an instruction may be issued to the examination technician to make the inquiry), and can accept a response as to whether or not the user can see the indicator (for example, when a Landolt ring is displayed, the direction of the opening is accepted, and when a character string is displayed, input of the character may be accepted). The visual acuity measurement unit 213 can also measure the visual acuity of the user by changing the size and displaying the indicator, and identifying the smallest size at which the user can see the indicator.

The visual acuity measurement unit 213 can display indicators with changed sizes at a predetermined distance (for example, 5 meters, etc.) from the user in the virtual space. The visual acuity measurement unit 213 displays images related to the virtual space on both the display devices 11 and 12 to provide a three-dimensional virtual space, and displays the indicator only on either the display device 11 or 12 corresponding to the eye to be measured while adjusting the image such that the indicator is arranged at a predetermined distance in the virtual space. This allows visual acuity to be measured for each eye.

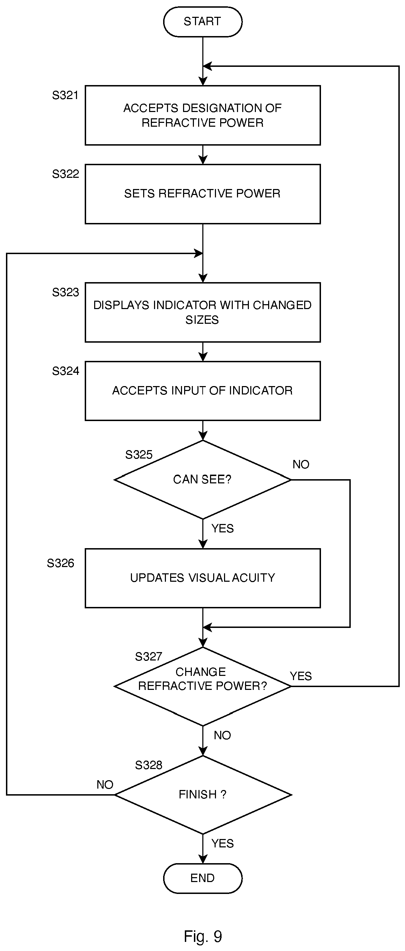

Fig. 9 is a diagram for explaining the operation of the computer 2 according to the second embodiment.

The computer 2 accepts, for example, designation of refractive power from an examination technician (S321). Note that the computer 2 may determine the refractive power. For example, the refractive power can be determined so as to be increased in order from a predetermined refractive power.

The computer 2 sets the refractive power of the HMD 1 (S322), provides a virtual space to the user by the display devices 11 and 12, and displays indicators with changed sizes on the display device 11 or 12 in front of either the left or right eye (S323). The computer 2 accepts input of the indicator (S324). For example, when the indicator is a Landolt ring, the direction of the opening can be accepted, and when the indicator is a character, the character can be accepted. The computer 2 determines whether or not the user can see the indicator based on whether or not the accepted content matches the displayed indicator (S325), and when the user can see the indicator (S325: YES), updates the visual acuity of the user according to the size of the indicator (S326).

The computer 2 accepts, for example, an instruction from an examination technician as to whether or not to change the refractive power, and when changing the refractive power (S327: YES), repeats the processing from step S321.

The computer 2 accepts, for example, an instruction from an examination technician as to whether or not to end the visual acuity examination, and when not ending (S328: NO), returns to step S323, and can display indicators with changed sizes.

As described above, according to the visual acuity measurement system of the second embodiment, corrected visual acuity can be easily measured.

Note that also in the second embodiment, the processing shown in Fig. 6 can be performed after setting the refractive power (S322).

Although the present embodiment has been described above, the above embodiment is for facilitating understanding of the present invention and is not for limiting interpretation of the present invention. The present invention can be changed and improved without departing from the spirit thereof, and the present invention also includes equivalents thereof.

### Disclosed Matters

Note that the present disclosure also includes the following configurations.

### Item 1

An information processing system comprising:
an image display unit configured to provide a virtual space to a user by displaying images on each of display devices corresponding to each of left and right eyes of the user;
an indicator display unit configured to arrange an indicator and a peripheral object in the virtual space at positions separated from the user, display the indicator only on the display device corresponding to one of the left and right eyes, display the peripheral object on the display devices corresponding to both the left and right eyes, and move the indicator to a distance at which the user can visually recognize the indicator; and a visual acuity measurement unit configured to determine visual acuity of one of the user's left and right eyes according to the distance.

### Item 2

The information processing system according to item 1, wherein:
the indicator display unit arranges a frame around the indicator, displays the frame on both the display devices corresponding to the left and right eyes, and moves the frame in accordance with movement of the indicator.

### Item 3

The information processing system according to item 1, wherein:
the indicator display unit changes a size of the indicator in the virtual space according to the distance such that a size of the indicator visually recognized by the user is constant.

### Item 4

The information processing system according to item 1, wherein
the indicator includes a first dotted line extending in a vertical direction and a second dotted line extending in a horizontal direction,
the indicator display unit deforms the indicator such that both the first and second dotted lines appear as dotted lines to the user when either the first or second dotted line appears as a solid line to the user, and
the visual acuity measurement unit determines astigmatism power of the user according to a degree of deformation of the indicator.

### Item 5

The information processing system according to item 4, wherein:
the indicator includes the first and second dotted lines and a third dotted line extending in a diagonal direction, and
the indicator display unit deforms the indicator such that all of the first through third dotted lines appear as dotted lines to the user when any of the first through third dotted lines appears as a solid line to the user.

### Item 6

An information processing method comprising steps of:
providing a virtual space to a user by displaying images on each of display devices corresponding to each of left and right eyes of the user;
arranging an indicator and a peripheral object in the virtual space at positions separated from the user, displaying the indicator only on the display device corresponding to one of the left and right eyes, displaying the peripheral object on the display devices corresponding to both the left and right eyes, and moving the indicator to a distance at which the user can visually recognize the indicator; and
determining visual acuity of one of the user's left and right eyes according to the distance, executed by a computer.

### Item 7

A program for causing a computer to execute steps of:
providing a virtual space to a user by displaying images on each of display devices corresponding to each of left and right eyes of the user;
arranging an indicator and a peripheral object in the virtual space at positions separated from the user, displaying the indicator only on the display device corresponding to one of the left and right eyes, displaying the peripheral object on the display devices corresponding to both the left and right eyes, and moving the indicator to a distance at which the user can visually recognize the indicator; and
determining visual acuity of one of the user's left and right eyes according to the distance.

### Reference Signs List

1 HMD
2 Computer

## Claims

1. An information processing system comprising:
an image display unit configured to display images on each of display devices corresponding to each of left and right eyes of a user to provide a virtual space to the user;
an indicator display unit configured to arrange an indicator and a peripheral object in the virtual space at positions separated from the user, display the indicator only on the display device corresponding to one of the left and right eyes, display the peripheral object on the display devices corresponding to both of the left and right eyes, and move the indicator to a distance at which the user can visually recognize the indicator; and
a visual acuity measurement unit configured to determine visual acuity of one of the user's left and right eyes according to the distance.

2. The information processing system according to claim 1, wherein:
the indicator display unit changes a size of the indicator in the virtual space according to the distance such that a size of the indicator visually recognized by the user is constant.

3. An information processing method executed by a computer, the method comprising:
displaying images on each of display devices corresponding to each of left and right eyes of a user to provide a virtual space to the user;
arranging an indicator and a peripheral object in the virtual space at positions separated from the user, displaying the indicator only on the display device corresponding to one of the left and right eyes, displaying the peripheral object on the display devices corresponding to both of the left and right eyes, and moving the indicator to a distance at which the user can visually recognize the indicator; and
determining visual acuity of one of the user's left and right eyes according to the distance.

4. A program for causing a computer to execute:
displaying images on each of display devices corresponding to each of left and right eyes of a user to provide a virtual space to the user;
arranging an indicator and a peripheral object in the virtual space at positions separated from the user, displaying the indicator only on the display device corresponding to one of the left and right eyes, displaying the peripheral object on the display devices corresponding to both of the left and right eyes, and moving the indicator to a distance at which the user can visually recognize the indicator; and
determining visual acuity of one of the user's left and right eyes according to the distance.
